(19) Europäisches Patentamt
European Patent Office
Office européen des brevets

(11) Veröffentlichungsnummer: **0 110 253**
**B1**

# (12) EUROPÄISCHE PATENTSCHRIFT

(45) Veröffentlichungstag der Patentschrift: **06.11.85**

(51) Int. Cl.⁴: **C 07 C 97/10**, C 07 D 295/10, A 61 K 31/135

(21) Anmeldenummer: **83111501.9**

(22) Anmeldetag: **17.11.83**

(54) **Substituierte 1-Oxo-2-phenyl-2-(2-alkylaminoethyl)-1,2,3,4-tetrahydronaphthaline, ihre Herstellung und Verwendung.**

(30) Priorität: **25.11.82 DE 3243518**

(43) Veröffentlichungstag der Anmeldung: **13.06.84 Patentblatt 84/24**

(45) Bekanntmachung des Hinweises auf die Patenterteilung: **06.11.85 Patentblatt 85/45**

(84) Benannte Vertragsstaaten: **AT BE CH DE FR GB IT LI NL SE**

(56) Entgegenhaltungen:
**DE - C - 728 103**
**GB - A - 1 021 185**

**JOURNAL OF MEDICINAL CHEMISTRY, Band 20, Nummer 5, 1977 W.M. WELCH et al. "Analgesic and Tranquilizing Activity of 5,8-Disubstituted 1-Tetralone Mannich Bases" Seiten 699-705**

(73) Patentinhaber: **BASF Aktiengesellschaft, Carl-Bosch-Strasse 38, D-6700 Ludwigshafen (DE)**

(72) Erfinder: **Seitz, Werner, Dr., Bismarckstrasse 22 B, D-6831 Plankstadt (DE)**
Erfinder: **Teschendorf, Hans-Juergen, Dr., Georg-Nuss-Strasse 5, D-6724 Dudenhofen (DE)**
Erfinder: **Michel, Alfred, Dr., Gruenstadter Strasse 52, D-6753 Enkenbach/Alsenborn (DE)**
Erfinder: **Traut, Martin, Dr., Muehltalstrasse 125, D-6900 Heidelberg (DE)**
Erfinder: **Hofmann, Hans Peter, Dr., Untere Hart 12, D-6703 Limburgerhof (DE)**
Erfinder: **Kreiskott, Horst, Prof. Dr., Am Boehlig, D-6706 Wachenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft neue substituierte 1-Oxo-2-phenyl-2-(2-alkylaminoethyl)-1,2,3,4-tetrahydronaphthaline, Verfahren zu deren Herstellung sowie diese Verbindungen zur Bekämpfung von Krankheiten.

Die zentrale (tranquilisierende, analgetische und neuroleptische) Wirkung substituierter 1-Oxo-2-dialkylaminomethyl-1,2,3,4-tetrahydro-naphtaline ist verschiedentlich beschrieben (z.B. J. Med. Chem. *20*, 699 (1977), Naunyn-Schmiedebergs Arch. Exp. Pathol. Pharmakol. *236*, 92 (1959), *238*, 114 (1960)).

Es wurden nun neue Verbindungen gefunden, die gute antidepressive Wirksamkeit zeigen.

Gegenstand der Erfindung sind 1-Oxo-2-phenyl-2-(2-alkylaminoethyl)-1,2,3,4-tetrahydronaphthaline der Formel I

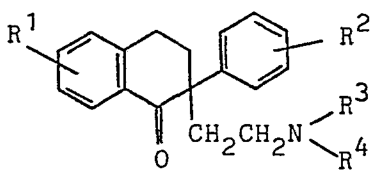
(I)

worin

$R^1$ und $R^2$ gleich oder verschieden sind und Wasserstoff- oder Halogenatome oder Trifluormethyl-, $C_{1-4}$-Alkyl oder $C_{1-4}$-Alkoxygruppen bedeuten,

$R^3$ eine $C_{1-6}$-Alkylgruppe ist und

$R^4$ ein Wasserstoffatom oder eine $C_{1-6}$-Alkyl- oder eine Benzylgruppe darstellt oder

$R^3$ und $R^4$ zusammen auch eine $C_{2-5}$-Alkylenkette bedeuten sowie deren Salze mit physiologisch verträglichen Säuren.

Als physiologisch verträgliche Säuren kommen beispielsweise in Frage: Salzsäure, Bromwasserstoffsäure, Schwefelsäure, Phosphorsäure, Amidosulfonsäure, Salpetersäure oder organische Säuren, wie Essigsäure, Propionsäure, Oxalsäure, Malonsäure, Bernsteinsäure, Fumarsäure, Maleinsäure, Zitronensäure, Weinsäure, Milchsäure.

$R^1$ und $R^2$ stellen vorzugsweise Wasserstoff- oder Chloratome dar, $R^3$ ist vorzugsweise eine Methylgruppe und $R^4$ vorzugsweise ein Wasserstoffatom oder eine Methylgruppe.

Die neuen Verbindungen lassen sich herstellen, indem man eine 2-Phenyl-2-(2-phenethyl)-4-dialkylaminobutansäure der Formel II

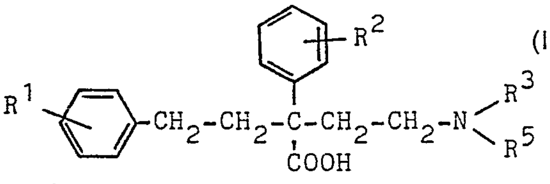
(II)

worin

$R^1$, $R^2$ und $R^3$ die angegebene Bedeutung haben und $R^5$ dasselbe wie $R^4$ ausgenommen ein Wasserstoffatom bedeutet, cyclisiert und in den so erhaltenen Verbindungen gewünschtenfalls den Rest $R^5$ gegen ein Wasserstoffatom austauscht und die so erhaltenen Verbindungen – falls gewünscht – in ihre Salze mit physiologisch verträglichen Säuren überführt.

Die Cyclisierung der 2-Phenyl-2-(2-phenethyl)-4-dialkylaminobutansäure II ist nach der klassischen Friedel-Crafts-Acylierung möglich. Als Acylierungskatalysatoren kommen auch Polyphosphorsäure, konz. Schwefelsäure, Methansulfonsäure, Gemische aus Methansulfonsäure und Phosphorpentoxid sowie Fluorwasserstoffsäure in Frage. Bevorzugt wird mit Methansulfonsäure oder Gemischen aus Methansulfonsäure und Phosphorpentoxid gearbeitet.

Die Reaktionstemperaturen für die Cyclisierungsreaktion sind von den eingesetzten Lewissäuren abhängig. Sie können von 0 bis 150° C variieren. Bei der Cyclisierung mit Methansulfonsäure wird ein Temperaturbereich von 80 bis 120° C, bei der Verwendung von Methansulfonsäure/Phosphorpentoxid-Gemischen 20 bis 50° C bevorzugt.

Die Darstellung der sekundären Aminoverbindungen der Formel I ($R^3$ = H, $R^4$ = Alkyl) erfolgt nach bekannten Verfahren durch Umsetzung der tertiären Amine der Formel I ($R^3$ und $R^4$ = Alkyl) mit Chlorameisensäureester zu Carbaminsäureester (Formel I, $R^3$ = Alkyl, $R^4$ = COOAlkyl) mit anschliessender Verseifung und Decarboxylierung (M.E. Jung, M.A. Lyster, J.C.S. Chem. Com. *1978*, 315; J.W. Barton in „Protective Groups in Organic Chemistry", Ed. J.F.W. McOmie, London 1973, 56-61).

Die Verbindungen weisen mindestens ein chirales Kohlenstoffatom auf, z.B. das Kohlenstoffatom 2 des 1,2,3,4-Tetrahydronaphthalinringes, das durch Aryl- und Dialkylaminoethylrest substituiert ist. Folglich können die Verbindungen I entweder in optisch aktiven Formen oder als racemische Mischungen hergestellt werden. Wenn erwünscht, können die Racemate der Verbindungen I durch herkömmliche Trenntechniken, z.B. durch Trennung (fraktionierte Kristallisation, Säulenchromatographie) der diastereomeren Salze, in ihre optischen Antipoden gespalten werden. Die diastereomeren Salze sind durch Umsetzung der Verbindungen I mit chiralen Säuren herstellbar.

Beispiele für chirale Säuren sind die optisch aktiven Formen der Campher-10-sulfonsäure, α-Bromcampher-π-sulfonsäure, Camphersäure, Menthoxyessigsäure, Weinsäure, Mandelsäure, O,O-Diacetyl, O,O-Dibenzoyl- und O,O-Di-4-toluolylweinsäure. Die getrennten, reinen diastereomeren Salze können dann nach Standardmethoden zu den optischen Isomeren gespalten werden. Die racemischen Verbindungen I bzw. II lassen sich auch auf chromatographischem Wege an optisch aktiven Trägermaterialien (z.B. acetylierte quervernetzte Cellulose) in ihre Antipoden trennen.

Die noch nicht beschriebenen 2-Phenyl-2-(2-phenethyl)-4-dialkylaminobutansäuren II lassen sich aus den entsprechenden Säurenitrilen (III) durch Verseifung der Nitrilgruppe beispielsweise mit konzentrierter Bromwasserstoffsäure oder mit Kaliumhydroxid in Alkohol bei erhöhter Temperatur darstellen.

Die Nitrile III lassen sich aus den entsprechenden 2,4-Diphenylbutannitrilen durch Umsetzen mit 2-Dialkylaminoethylchlorid nach einem phasentransferkatalysierten Verfahren herstellen.

Die 2,4-Diphenylbutannitrile sind nach J. Chem. Soc. 1956, 691 zugänglich.

Zur Herstellung der optisch aktiven Formen der Verbindungen I kann man bereits von optisch aktiven Vorstufen II bzw. III ausgehen. Hierzu werden die Verbindungen II bzw. III nach den oben angegebenen Methoden in ihre optisch aktiven Formen übergeführt. Die Verbindungen II können jedoch auch mit chiralen Basen, z.B. α-Phenethylaminen, Brucin, Cinchonidin, Cinchonin, Strychnin, Chinin und Chinidin, in ihre optischen Antipoden getrennt werden.

Die erfindungsgemässen Verbindungen und ihre Salze mit physiologisch verträglichen Säuren eignen sich zur Pharmakotherapie von psychischen Störungen, insbesondere von Depressionen. Die antidepressive Wirkung der erfindungsgemässen Substanzen wurde im folgenden Modell untersucht:

Reserpin (2,15 mg/kg subkutan) verursacht an Mäusen (Stamm: Swiss; männlich, 20-26 g Gewicht) eine Senkung der Körpertemperatur um durchschnittlich 3° C, gemessen zwei Stunden nach Reserpingabe und bei einer Umgebungstemperatur von 20-22° C. Antidepressiva hemmen diese Hypothermie dosisabhängig. Die Prüfsubstanzen wurden oral 60 min vor der Reserpingabe appliziert.

Als $ED_{50}$ wird aus der linearen Regression zwischen log Dosis (mg/kg) und relativer Abnahme der Hypothermie die Dosis ermittelt, welche die durch die Reserpin induzierte Hypothermie um 50% hemmt.

Die Ergebnisse der Prüfung sind in Tabelle I zusammengestellt. Die $ED_{50}$-Werte der erfindungsgemässen Verbindungen liegen überwiegend unter der $ED_{50}$, die für das Standard-Antidepressivum Imipramin bestimmt wurde. Die Substanzen sind somit wirksamer als die Vergleichssubstanz. Die Wirksamkeit des Imipramin wird zum Teil deutlich (bei Beispiel 18c um das 72fache) übertroffen.

*Tabelle 1*

| Substanz des Beispiels Nr. | Antidepressive Wirkung $ED_{50}$ mg/kg p.o. |
|---|---|
| 1 | 0,14 |
| 2 | 6,8 |
| 3 | 4,6 |
| 5 | 4,9 |
| 8 | 5,2 |
| 12 | 0,9 |
| 14 | 2,3 |
| 18a | 0,85 |
| 18c | 0,09 |
| Imipramin | 6,8 |

Das Wirkungsspektrum der neuen Substanzen wird weiter durch eine starke Hemmung der Wiederaufnahme biogener Amine – vor allem Noradrenalin und Serotonin – in Synaptosomen bestimmt.

Die neuen Verbindungen können in üblicher Weise oral oder parenteral (subkutan, intravenös, intramuskulär, intraperitoneal) verabfolgt werden.

Die Dosierung hängt vom Alter, Zustand und Gewicht des Patienten sowie von der Applikationsform ab. In der Regel beträgt die tägliche Wirkstoffdosis zwischen etwa 0,01 bis 10 mg/kg Körpergewicht sowohl bei intravenöser, subkutaner oder intramuskulärer als auch bei oraler Anwendung.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Lösungen oder Suppositorien. Diese werden in üblicher Weise hergestellt. Die Wirkstoffe können dabei mit den gebräuchlichen galenischen Hilfsmitteln, wie Tablettenbindern, Füllstoffen, Konservierungsmitteln, Tablettensprengmitteln, Fliessregulierungsmitteln, Weichmachern, Netzmitteln, Dispergiermitteln, Emulgatoren, Lösungsmitteln, Retardierungsmitteln und/ oder Antioxidantien verarbeitet werden (vgl. H. Sucker et al: Pharmazeutische Technologie, Thieme-Verlag, Stuttgart 1978). Die so erhaltenen Zubereitungen enthalten den Wirkstoff normalerweise in einer Menge von 0,1 bis 99 Gew.%.

Die Erfindung wird nachstehend anhand von Beispielen erläutert.

I. Herstellung der Ausgangsmaterialien

A. Nitrile der Säuren der Formel II

a) 24 g 2-Dimethylaminoethylchlorid-hydrochlorid (0,17 Mol) wurden in 30 ml Wasser gelöst und unter Rühen mit 30 ml 50%iger Kaliumhydroxidlösung versetzt. Die abgeschiedene Base wurde in 70 ml Toluol aufgenommen und über wasserfreiem Kaliumcarbonat getrocknet. Die toluolische Lösung wurde in einem 500-ml-Dreihalskolben überführt. Dazu gibt man 54 g 85%iges Kaliumhydroxidpulver, 6 g Kaliumcarbonat und 0,6 g Tetrabutylammoniumiodid. Unter Rühren wurden bei Raumtemperatur beginnend, 33,2 g 2,4-Diphenylbutannitril (0,15 Mol) in 30 ml Toluol gelöst zugetropft. Nach beendeter Zugabe wird 2 h bei 80° C nachgerührt.

Die erkaltete Reaktionslösung wurde in 300 ml Wasser gegossen, mit 300 ml n-Hexan versetzt und anschliessend die organische Phase abgetrennt. Diese wurde mehrmals mit wässeriger Kochsalzlösung gewaschen und das Lösungsmittel nach Trocknen über Kaliumcarbonat abdestilliert. Nach Lösen des Rückstandes in 300 ml Essigester fällte man das Hydrochlorid mit ethanolischer Salzsäure aus. Nach Kristallisation aus Isopropanol erhielt man 37 g (75%) 2-Phenyl-2-(2-phenethyl)-4-dimethylamino-butannitril-hydrochlorid, Fp. 214-216° C.

In anologer Weise wurden erhalten:

b) 2-(3-Tolyl)-2-(2-phenethyl)-4-dimethyla mino-butannitril-hydrochlorid, Fp. 208 bis 210° C,

c) 2-(4-Chlorphenyl)-2-(2-phenethyl)-4-di-

methylamino-butannitril-hydrochlorid, Fp. 223 bis 225° C,

d) 2-(4-Tolyl)-2-(2-phenethyl)-4-dimethylamino-butannitril-hydrochlorid, Fp. 229 bis 232° C,

e) 2-(4-Fluorphenyl)-2-(2-phenethyl)-4-dimethylamino-butannitril-hydrochlorid, Fp. 220 bis 221° C,

f) 2-(2-Chlorphenyl)-2-(2-phenethyl)-4-dimethylamino-butannitril-hydrochlorid, Fp. 220 bis 221° C,

g) 2-(3-Trifluormethylphenyl)-2-(2-phenethyl)-4-dimethylamino-butannitril-hydrochlorid, Fp. 119 bis 200° C,

h) 2-(3-Chlorphenyl)-2-(2-phenethyl)-4-dimethylamino-butannitril-hydrochlorid, Fp. 215 bis 217° C,

i) 2-Phenyl-2-(2-phenethyl)-4-diethylamino-butannitril-hydrochlorid, Fp. 208 bis 210° C,

j) 2-Phenyl-2-(2-phenethyl)-4-(1-pyrrolidinyl)-butannitril-hydrochlorid, Fp. 228 bis 230° C,

k) 2-Phenyl-2-(2-phenethyl)-4-(1-piperidinyl)-butannitril-hydrochlorid, Fp. 226 bis 227° C,

l) 2-Phenyl-2-[2-(2-chlorphenyl)ethyl]-4-dimethylamino-butannitril, Fp. 219 bis 221° C,

m) 2-Phenyl-2-(2-phenethyl)-4-(N-isopropyl-N-benzyl)amino-butannitril, Fp. 170 bis 172° C,

n) 2-(2-Fluorphenyl)-2-(2-phenethyl)-4-dimethylamino-butannitril-hydrochlorid, Fp. 198 bis 199° C,

o) 2-(3-Methoxyphenyl)-2-(2-phenethyl)-4-dimethylamino-butannitril, Fp. 55 bis 57° C.

B. Säuren der Formel II

a) 82,2 g (0,25 Mol) 2-Phenyl-2-(2-phenethyl)-4-dimethylamino-butannitril-hydrochlorid wurden in 400 ml 48%iger Bromwasserstoffsäure gelöst und 15 h unter Rückfluss gekocht. Nach Abdestillieren der Bromwasserstoffsäure im Vakuum löste man den Rückstand in 2N-NaOH, extrahierte mehrmals mit Ether und stellte anschliessend die alkalische Lösung mit Eisessig auf pH 6 ein. Die ausgefallene Säure wurde abgesaugt, mit Wasser gewaschen und getrocknet. Ausbeute: 70,1 g (90%) 2-Phenyl-2-(2-phenethyl)-4-dimethylamino-butansäure. Eine Probe der Säure wurde aus einem Acetonitril-Wasser-Gemisch (7/1) umkristallisiert und zeigte den Schmelzpunkt 223 bis 225° C. Die Hauptmenge der Säure wurde ohne weitere Reinigung für die Cyclisierung eingesetzt.

In analoger Weise wurden erhalten:

b) 2-(3-Tolyl)-2-(2-phenethyl)-4-dimethylamino-butansäure,

c) 2-(4-Chlorphenyl)-2-(2-phenethyl)-4-dimethylamino-butansäure,

d) 2-(4-Tolyl)-2-(2-phenethyl)-4-dimethylamino-butansäure, Fp. 195 bis 196° C,

e) 2-(4-Fluorphenyl)-2-(2-phenethyl)-4-dimethylamino-butansäure, Fp. 218 bis 220° C,

f) 2-(2-Chlorphenyl)-2-(2-phenethyl)-4-dimethylamino-butansäure,

g) 2-(3-Trifluormethylphenyl)2-(2-phenethyl)-4-dimethylamino-butansäure, Fp. 172 bis 174° C,

h) 2-(3-Chlorphenyl)-2-(2-phenethyl)-4-dimethylamino-butansäure, Fp. 209 bis 211° C,

i) 2-Phenyl-2-(2-phenethyl)-4-diethylamino-butansäure,

j) 2-Phenyl-2-(2-phenethyl)-4-(1-pyrrolidinyl)-butansäure,

k) 2-Phenyl-2-(2-phenethyl)-4-(1-piperidinyl)-butansäure,

l) 2-Phenyl-2-[2-(2-chlorphenyl)-ethyl]-4-dimethylamino-butansäure, Fp. 176 bis 179° C,

m) 2-Phenyl-2-(2-phenethyl)-4-(N-isopropyl-N-benzyl)amino-butansäure,

n) 2-(2-Fluorphenyl)-2-(2-phenethyl)-4-dimethylamino-butansäure, Fp. 199° C,

o) 2-(3-Methoxyphenyl)-2-(2-phenethyl)-4-dimethylamino-butansäure.

II. Herstellung der Endprodukte

*Beispiel 1*

62,3 g (0,2 Mol) 2-Phenyl-2-(2-phenethyl)-4-dimethylamino-butansäure wurden in 200 ml einer Mischung aus 30 g Phosphorpentoxid und 350 g Methansulfonsäure bei Raumtemperatur gelöst und unter Rühren 20 h bei 40° C gehalten. Das erkaltete Reaktionsgemisch wurde auf Eis gegossen, mit Natronlauge alkalisch gemacht und die abgeschiedene Base mit Ether extrahiert. Die Etherphase wurde mehrmals mit Kochsalzlösung gewaschen, mit Magnesiumsulfat getrocknet und anschliessend der Ether abdestilliert. Nach Umkristallisation des Rückstandes aus einem Ether-Hexan-Gemisch (1:1) wurden 50 g (85%) 1-Oxo-2-phenyl-2-(2-dimethylaminoethyl)-1,2,3,4-tetrahydronaphthalin, Fp. 71 bis 72° C, isoliert.

Zur Herstellung des Hydrochlorids wurde die Base in Essigester gelöst und das Salz mit ethanolischer Salzsäure ausgefällt. Das Hydrochlorid hat nach Umkristallisation aus Ethanol einen Schmelzpunkt von 214 bis 216° C.

Ebenso wurden erhalten:

2. 1-Oxo-2-(3-tolyl)-2-(2-dimethylaminoethyl)-1,2,3,4-tetrahydronaphthalin-hydrochlorid, Fp. 232-234° C,

3. 1-Oxo-2-(4-chlorphenyl)-2-(2-dimethylaminoethyl)-1,2,3,4-tetrahydronaphthalin, Fp. 113-115° C,

4. 1-Oxo-2-(4-tolyl)-2-(2-dimethylaminoethyl)-1,2,3,4-tetrahydronaphthalin-hydrochlorid, Fp. 239-243° C,

5. 1-Oxo-2-(4-fluorphenyl)-2-(2-dimethylaminoethyl)-1,2,3,4-tetrahydronaphthalin-hydrochlorid, Fp. 270-273° C,

6. 1-Oxo-2-(2-chlorphenyl)-2-(2-dimethylaminoethyl)-1,2,3,4-tetrahydronaphthalin-hydrochlorid,

7. 1-Oxo-2-(3-trifluormethylphenyl)-2-(2-dimethylaminoethyl)-1,2,3,4-tetrahydronaphthalin-hydrochlorid, Fp. 198-201° C,

8. 1-Oxo-2-(3-chlorphenyl)-2-(2-dimethylaminoethyl)-1,2,3,4-tetrahydronaphthalin-hydrochlorid, Fp. 227-228° C,

9. 1-Oxo-2-phenyl-2-(2-diethylaminoethyl)-1,2,3,4-tetrahydronaphthalin-hydrochlorid, Fp. 218-220° C,

10. 1-Oxo-2-phenyl-2-[2-(1-pyrrolidinyl)ethyl]-1,2,3,4-tetrahydronaphthalin-hydrochlorid, Fp. 236-238° C,

11. 1-Oxo-2-phenyl-2[2-(1-piperidinyl)ethyl]-1,2,3,4-tetrahydronaphthalin-hydrochlorid, Fp. 239-242° C,

12. 1-Oxo-5-chlor-2-phenyl-2-(2-dimethylaminoethyl)-1,2,3,4-tetrahydronaphthalin, Fp. 107-109° C,

13. 1-Oxo-2-(2-fluorphenyl)-2-(2-dimethylaminoethyl)-1,2,3,4-tetrahydronaphthalin-hydrochlorid, Fp. 179-180° C,

14. 1-Oxo-2-(3-methoxyphenyl)-2-(2-dimethylaminoethyl)-1,2,3,4-tetrahydronaphthalin-hydrogenoxalat, Fp. 141-143° C.

*Beispiel 15*

1-Oxo-2-phenyl-2-(2 - methylaminoethyl)-1,2,3,4-tetrahydronaphthalin

29,3 g (0,1 Mol) 1-Oxo-2-phenyl-2-(2-dimethylaminoethyl) - 1,2,3,4-tetrahydronaphthalin (Beispiel 1) und 16,3 g (0,15 Mol) Chlorameisensäureethylester wurden in 200 ml Toluol gelöst und 5 h am Rückfluss gekocht. Nach Erkalten wurde die Toluollösung mit 5%iger Salzsäure und Kochsalzlösung gewaschen, über Natriumsulfat getrocknet und zur Trockne eingeengt.

Der Rückstand wurde in 100 ml trockenem Chloroform gelöst und mit 14 ml (0,1 Mol) Trimethylsilyliodid versetzt. Nach 3 h Kochen unter Rückfluss wurden 100 ml methanolischer Salzsäure zugesetzt und weitere 10 min unter Rückfluss erhitzt. Nach Abdestillieren des Lösungsmittels versetzte man den Rückstand mit Wasser, machte mit 2 N Natronlauge alkalisch und extrahierte die abgeschiedene Base mit Ether. Nach Waschen und Trocknen der etherischen Lösung wurde mit ethanolischer Salzsäure das Hydrochlorid ausgefällt und aus Isopropanol umkristallisiert. Man erhielt 22,1 g (70%) 1-Oxo-2-phenyl-2-(2-methylaminoethyl) - 1,2,3,4-tetrahydronaphthalin-hydrochlorid, Fp. 152-154° C.

Ebenso wurden erhalten:

16. 1-Oxo-2-phenyl-2-(2-isopropylaminoethyl)-1,2,3,4-tetrahydronaphthalin-hydrochlorid, Fp. 187-189° C.

17. 1-Oxo-2-phenyl-2-(2-ethylaminoethyl)-1,2,3,4-tetrahydronaphthalin-hydrochlorid, Fp. 183-185° C.

*Beispiel 18*

Racematspaltung von 1-Oxo-2-phenyl-2-(2-dimethylaminoethyl)-1,2,3,4-tetrahydronaphthalin

a) Rechtsdrehender Antipode

29,3 g (0,1 Mol) 1-Oxo-2-phenyl-2-(2-dimethylaminoethyl)-1,2,3,4-tetrahydronaphthalin (Beispiel 1) und 20,2 g (0,05 Mol) (−)-O,O'-Di-t-toluoyl-L-weinsäure wurden in 400 ml Ethanol gelöst. Nach 5 min wurde das Kristallisat abgesaugt und zweimal aus Methanol umkristallisiert. Der gefundene Drehwert von $[\alpha]_{589}^{20}$ = +35,8° (Methanol, c = 10 mg/ml) veränderte sich durch nochmalige Kristallisation nicht. Die aus dem Salz freigesetzte Base hat den Schmelzpunkt 64 bis 66° C und einen Drehwert $[\alpha]_{589}^{20}$ = +249° (Methanol, c = 10 mg/ml).

Der Schmelzpunkt des Hydrochlorids beträgt 231 bis 232° C und sein Drehwert $[\alpha]_{589}^{20}$ = +238° (Methanol, c = 10 mg/ml).

c) Linksdrehender Antipode

Analog a) wurde durch Umsetzung mit (+)-O,O'-Di-4-toluoyl-D-weinsäure der linksdrehenden Antipode erhalten.

Base: Fp. 64-66° C
$[\alpha]_{589}^{20}$ = −246° (Methanol, c = 10 mg/ml)

Hydrochlorid: Fp. 231-232° C
$[\alpha]_{589}^{20}$ = −238° (Methanol, c = 10 mg/ml)

Galenische Beispiele:

*Beispiel A*

Auf einer Tablettenpresse wurden in üblicher Weise Tabletten folgender Zusammensetzung gepresst:

10 mg 1-Oxo-2-phenyl-2-(2-dimethylaminoethyl)-1,2,3,4-tetrahydronaphthalin-hydrochlorid
50 mg Maisstärke
4,5 mg Gelatine
15 mg Milchzucker
7,5 mg Talk
0,75 mg Aerosil® (chemisch reine Kieselsäure in submikroskopisch feiner Verteilung)
2,25 mg Kartoffelstärke (als 6%iger Kleister)

*Beispiel B*

In üblicher Weise wurden Dragees folgender Zusammensetzung hergestellt:

10 mg 1-Oxo-2-phenyl-2-(2-dimethylaminoethyl)-1,2,3,4-tetrahydronaphthalin-hydrochlorid
50 mg Kernmasse
40 mg Verzuckerungsmasse

Die Kernmasse bestand aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschliessend mit einem magensaftresistenten Überzug versehen.

*Beispiel C*

Es wurden 5,0 g 1-Oxo-2-phenyl-2-(2-dimethylaminoethyl) - 1,2,3,4-tetrahydronaphthalin-hydrochlorid in 2,0 l Wasser gelöst, mit Kochsalz isotonisch eingestellt und in 2 ml fassende Ampullen steril abgefüllt.

**Patentansprüche** für die Vertragsstaaten BE, CH, DE, FR, GB, IT, LI, NL, SE

1. 1-Oxo-2-phenyl-2-(2-alkylaminoethyl)-1,2,3,4-tetrahydronaphthaline der Formel I

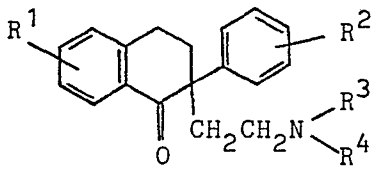

(I)

worin

$R^1$ und $R^2$ gleich oder verschieden sind und Wasserstoff- oder Halogenatome oder Trifluormethyl-, $C_{1-4}$-Alkyl oder $C_{1-4}$-Alkoxygruppen bedeuten,

$R^3$ eine $C_{1-6}$-Alkylgruppe ist und

$R^4$ ein Wasserstoffatom oder eine $C_{1-6}$-Alkyl- oder eine Benzylgruppe darstellt oder

$R^3$ und $^4$ zusammen auch eine $C_{2-5}$-Alkylenkette bedeuten

sowie deren Salze mit physiologisch verträglichen Säuren.

2. 1-Oxo-2-phenyl-2-(2-dimethylaminoethyl)-1,2,3,4-tetrahydronaphthalin.

3. Verbindungen der Formel I gemäss Anspruch 1 in Form ihrer linksdrehenden Antipoden.

4. (−)-1-Oxo-2-phenyl-2-(2-dimethylaminoethyl)-1,2,3,4-tetrahydronaphthalin.

5. Verbindungen der Formel I gemäss Anspruch 1 in Form ihrer rechtsdrehenden Antipoden.

6. (+)-1-Oxo-2-phenyl-2-(2-dimethylaminoethyl)-1,2,3,4-tetrahydronaphthalin.

7. Verfahren zur Herstellung der Verbindungen der Formel I gemäss Anspruch 1, dadurch gekennzeichnet, dass man eine 2-Phenyl-2-(2-phenethyl)-4-dialkylamino-butan-säure der Formel II

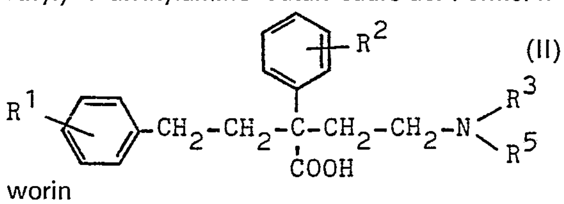

(II)

worin

$R^1$, $R^2$ und $R^3$ die angegebene Bedeutung haben und $R^5$ dasselbe wie $R^4$ ausgenommen ein Wasserstoffatom bedeutet, cyclisiert und in den so erhaltenen Verbindungen gewünschtenfalls den Rest $R^5$ gegen ein Wasserstoffatom austauscht und die so erhaltenen Verbindungen – falls gewünscht – in ihre Salze mit physiologisch verträglichen Säuren überführt.

8. Arzneimittel enthaltend eine Verbindung gemäss Anspruch 1.

9. Verbindung der Formel I gemäss Anspruch 1 zur Verwendung bei der Bekämpfung von Krankheiten.

**Patentansprüche** für den Vertragsstaat: AT

1. Verfahren zur Herstellung von 1-Oxo-2-phenyl-2-(2-alkylaminoethyl)-1,2,3,4-tetrahydronaphthaline der Formel I

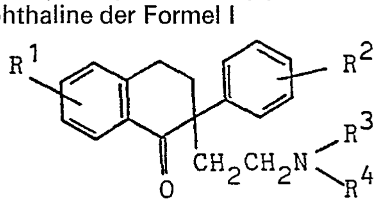

(I)

worin

$R^1$ und $R^2$ gleich oder verschieden sind und Wasserstoff- oder Halogenatome oder Trifluormethyl-, $C_{1-4}$-Alkyl oder $C_{1-4}$-Alkoxygruppen bedeuten,

$R^3$ eine $C_{1-6}$-Alkylgruppe ist und

$R^4$ ein Wasserstoffatom oder eine $C_{1-6}$-Alkyl- oder eine Benzylgruppe darstellt oder

$R^3$ und $R^4$ zusammen auch eine $C_{2-5}$-Alkylenkette bedeuten

sowie deren Salze mit physiologisch verträglichen Säuren, dadurch gekennzeichnet, dass man eine 2-Phenyl-2-(2-phenethyl)-4-dialkylaminobutansäure der Formel II

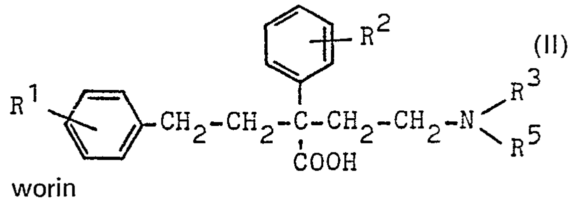

(II)

worin

$R^1$, $R^2$ und $R^3$ die angegebene Bedeutung haben und $R^5$ dasselbe wie $R^4$ ausgenommen ein Wasserstoffatom bedeutet, cyclisiert und in den so erhaltenen Verbindungen gewünschtenfalls den Rest $R^5$ gegen ein Wasserstoffatom austauscht und die so erhaltenen Verbindungen – falls gewünscht – in ihre Salze mit physiologisch verträglichen Säuren überführt.

2. Verfahren gemäss Anspruch 1, dadurch gekennzeichnet, dass man 1-Oxo-2-phenyl-2-(2-dimethylaminoethyl)-1,2,3,4-tetrahydronaphthalin herstellt.

3. Verfahren gemäss Anspruch 1, dadurch gekennzeichnet, dass man die Verbindungen der Formel I in Form ihrer linksdrehenden Antipoden herstellt.

4. Verfahren gemäss Anspruch 1, dadurch gekennzeichnet, dass man (−)-1-Oxo-2-phenyl-2-(2-dimethylaminoethyl)-1,2,3,4-tetrahydronaphthalin herstellt.

5. Verfahren gemäss Anspruch 1, dadurch gekennzeichnet, dass man die Verbindungen der Formel I in Form ihrer rechtsdrehenden Antipoden herstellt.

6. Verfahren gemäss Anspruch 1, dadurch gekennzeichnet, dass man (+)-1-Oxo-2-phenyl-2-(dimethylaminoethyl)-1,2,3,4-tetrahydronaphthalin herstellt.

**Claims** for the Contracting States BE, CH, DE, FR, GB, IT, LI, NL, SE

1. A 1-oxo-2-phenyl-2-(2-alkylaminoethyl)-1,2,3,4-tetrahydronaphthalene of the formula I

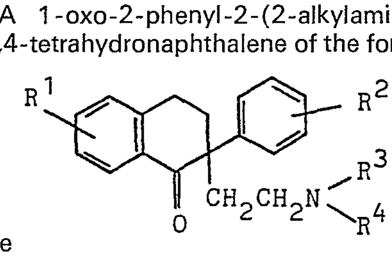

(I)

where

$R^1$ and $R^2$ are identical or different and are each hydrogen, halogen, trifluoromethyl, $C_1$-$C_4$-alkyl

or $C_1$-$C_4$-alkoxy, $R^3$ is $C_1$-$C_6$-alkyl and $R^4$ is hydrogen, $C_1$-$C_6$-alkyl or benzyl, or $R^3$ and $R^4$ together may furthermore be a $C_2$-$C_5$-alkylene chain, and its salts with physiologically tolerated acids.

2. 1-Oxo-2-phenyl-2-(2-dimethylaminoethyl)-1,2,3,4-tetrahydronaphthalene.

3. A compound of the formula I as claimed in claim 1, in the form of its levorotatory antipode.

4. (−)-1-Oxo-2-phenyl-2-(2-dimethylaminoethyl)-1,2,3,4-tetrahydronaphthalene.

5. A compound of the formula I as claimed in claim 1, in the form of its dextrorotatory antipode.

6. (+)-1-Oxo-2-phenyl-2-(2-dimethylaminoethyl)-1,2,3,4-tetrahydronaphthalene.

7. A process for the preparation of a compound of the formula I as claimed in claim 1, wherein a 2-phenyl-2-(2-phenethyl)-4-dialkylaminobutanoic acid of the formula II

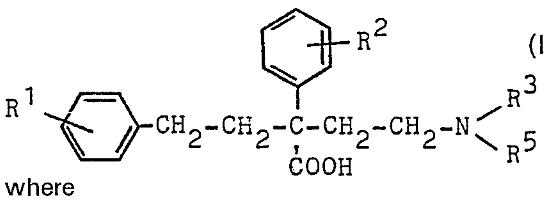

(II)

where

$R^1$, $R^2$ and $R^3$ have the above meanings and $R^5$ has the same meanings as $R^4$, with the exception of hydrogen, is subjected to a cyclization reaction, and, if desired, the radical $R^5$ in the resulting compound is exchanged for a hydrogen atom, and, if desired, the resulting compound is converted into its salts with physiologically tolerated acids.

8. A pharmaceutical containing a compound as claimed in claim 1.

9. A compound of the formula I as claimed in claim 1 for use in the treatment of disorders.

**Claims** for the Contracting State: AT

1. A process for the preparation of a 1-oxo-2-phenyl-2-(2-alkylaminoethyl)-1,2,3,4-tetrahydronaphthalene of the formula I

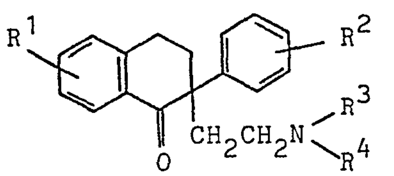

(I)

where

$R^1$ and $R^2$ are identical or different and are each hydrogen, halogen, trifluoromethyl, $C_1$-$C_4$-alkyl or $C_1$-$C_4$-alkoxy, $R^3$ is $C_1$-$C_6$-alkyl and $R^4$ is hydrogen, $C_1$-$C_6$-alkyl or benzyl, or $R^3$ and $R^4$ together may furthermore be a $C_2$-$C_5$-alkylene chain, and its salts with physiologically tolerated acids, wherein a 2-phenyl-2-(2-phenethyl)-4-dialkylaminobutanoic acid of the formula II

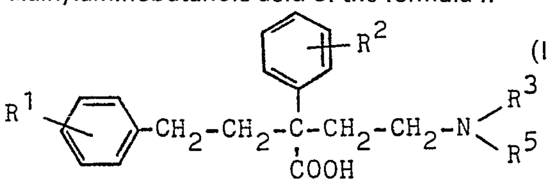

(II)

where

$R^1$, $R^2$ and $R^3$ have the above meanings and $R^5$ has the same meanings as $R^4$, with the exception of hydrogen, is subjected to a cyclization reaction, and, if desired, the radical $R^5$ in the resulting compound is exchanged for a hydrogen atom, and, if desired, the resulting compound is converted into its salts with physiologically tolerated acids.

2. A process as claimed in claim 1, wherein 1-oxo-2-phenyl-2-(2-dimethylaminoethyl)-1,2,3,4-tetrahydronaphthalene is prepared.

3. A process as claimed in claim 1, wherein the compound of the formula I is prepared in the form of its levorotatory antipode.

4. A process as claimed in claim 1, wherein (−)-1-oxo-2-phenyl-2-(2-dimethylaminoethyl)-1,2,3,4-tetrahydronaphthalene is prepared.

5. A process as claimed in claim 1, wherein the compound of the formula I is prepared in the form of its dextrorotatory antipode.

6. A process as claimed in claim 1, wherein (+)-1-oxo-2-phenyl-2-(2-dimethylaminoethyl)-1,2,3,4-tetrahydronaphthalene is prepared.

**Revendications** pour les Etats contractants BE, CH, DE, FR, GB, IT, LI, NL, SE

1. 1-oxo-2-phényl-2-(2-alkylaminoéthyl)-1,2,3,4-tétrahydronaphthalènes de formule I

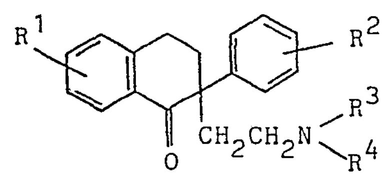

(I)

dans laquelle

$R^1$ et $R^2$ sont identiques ou différents et représentent des atomes d'hydrogène ou d'halogène, ou des groupes trifluorométhyle, alkyle en $C_{1-4}$, ou alcoxy en $C_{1-4}$

$R^3$ est un groupe alkyle en $C_{1-6}$ et

$R^4$ représente un atome d'hydrogène ou un groupe alkyle en $C_{1-6}$ ou benzyle, ou

$R^3$ et $R^4$ représentent ensemble aussi une chaîne alkylène en $C_{2-5}$

ainsi que leurs sels d'acides physiologiquement acceptables.

2. 1-oxo-2-phényl-2-(2-diméthylaminoéthyl)-1,2,3,4-tétrahydronaphtalène.

3. Composés de formule I selon la revendication 1 sous forme de leurs antipodes lévogyres.

4. (−)-1-oxo-2-phényl-2-(2-diméthylaminoéthyl)-1,2,3,4-tétrahydronaphtalène.

5. Composés de formule I selon la revendication 1 sous forme de leurs antipodes dextrogyres.

6. (+)-1-oxo-2-phényl-2-(2-diméthylaminoéthyl)-1,2,3,4-tétrahydronaphtalène.

7. Procédé de préparation des composés de formule I selon la revendication 1 caractérisé par le fait qu'on cyclise un acide 2-phényl-2-(2-phényléthyl)-4-dialkylaminobutanoïque de formule II

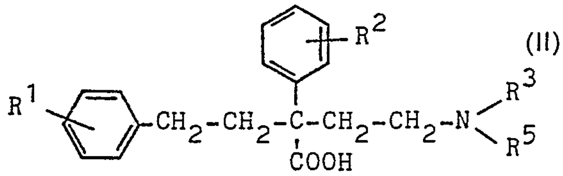

(II)

où

$R^1$, $R^2$ et $R^3$ ont les significations indiquées et $R^5$ les mêmes que $R^4$ à l'exclusion d'un atome d'hydrogène et, dans les composés ainsi obtenus, on échange, si on le souhaite, le reste $R^5$ contre un atome d'hydrogène et l'on transforme, si on le souhaite, les composés ainsi obtenus en leurs sels d'acides acceptables physiologiquement.

8. Médicament contenant un composé selon la revendication 1.

9. Composé de formule I selon la revendication 1 pour l'utilisation dans la lutte contre les maladies.

**Revendications** pour l'Etat contractant: AT

1. Procédé de préparation de 1-oxo-2-phényl-2-(2-alkylaminoéthyl)-1,2,3,4-tétrahydronaphtalènes de formule I

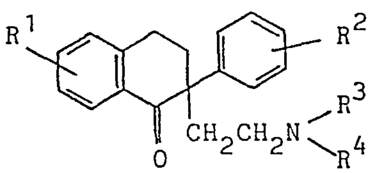

(I)

dans laquelle

$R^1$ et $R^2$ sont identiques ou différents et représentent des atomes d'hydrogène ou d'halogène, ou des groupes trifluorométhyle, alkyle en $C_{1-4}$, ou alcoxy en $C_{1-4}$

$R^3$ est un groupe alkyle en $C_{1-6}$ et

$R^4$ représente un atome d'hydrogène ou un groupe alkyle en $C_{1-6}$ ou benzyle, ou

$R^3$ et $R^4$ représentent ensemble aussi une chaîne alkylène en $C_{2-5}$

ainsi que leurs sels d'acides physiologiquement acceptables, caractérisé par le fait qu'on cyclise un acide 2-phényl-2-(2-phényléthyl)-4-dialkylaminobutanoïque de formule II

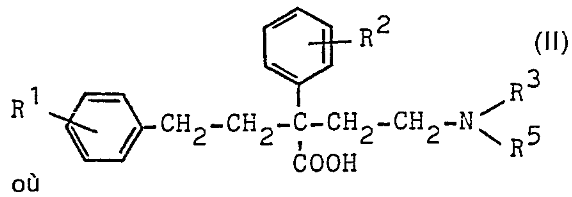

(II)

où

$R^1$, $R^2$ et $R^3$ ont les significations indiquées et $R^5$ les mêmes que $R^4$ à l'exclusion d'un atome d'hydrogène et, dans les composés ainsi obtenus, on échange, si on le souhaite, le reste $R^5$ contre un atome d'hydrogène et l'on transforme, si on le souhaite, les composés ainsi obtenus en leurs sels d'acides acceptables physiologiquement.

2. Procédé selon la revendication 1 caractérisé par le fait qu'on prépare 1-oxo-2-phényl-2-(2-diméthylaminoéthyl)-1,2,3,4-tétrahydronaphtalène.

3. Procédé selon la revendication 1, caractérisé par le fait qu'on prépare les composés de formule I sous forme de leurs antipodes levogyres.

4. Procédé selon la revendication 1, caractérisé par le fait qu'on prépare le (−)-1-oxo-2-phényl-2-(2-diméthylaminoéthyl)-1,2,3,4-tétrahydronaphtalène.

5. Procédé selon la revendication 1, caractérisé par le fait qu'on prépare les composés de formule I sous forme de leurs antipodes dextrogyres.

6. Procédé selon la revendication 1, caractérisé par le fait qu'on prépare la (+)-1-oxo-2-phényl-2-(2-diméthylaminoéthyl)-1,2,3,4-tétrahydronaphtalène.